# EUROPEAN PATENT APPLICATION

(11) **EP 3 695 780 A1**
(43) Date of publication of application: **19.08.2020**
(21) Application number: 18865973.4
(22) Date of filing: 12.09.2018
(51) Int. Cl.: A61B 5/0408, A61B 5/0478, A61B 5/0492

(54) **BRAIN WAVE DETECTION BIOELECTRODE**

(30) Priority: 10.10.2017 JP 2017197153
(71) Applicant: NOK Corporation, Minato-ku Tokyo 105-8585 (JP)
(72) Inventor: NOMURA Yu, Fujisawa-shi Kanagawa 251-0042 (JP); FUTASHIMA Ryo, Fujisawa-shi Kanagawa 251-0042 (JP); UDA Toru, Fujisawa-shi Kanagawa 251-0042 (JP)
(74) Representative: TBK
(86) International application number: PCT/JP2018/033757
(87) International publication number: WO 2019/073740

(57) **Abstract**

A brain wave detection bioelectrode capable of improving adhesion is provided. The brain wave detection bioelectrode (1) includes: a holder part (10) that includes a plurality of through holes (11); an electrode part (20) that is formed of at least one conductive elastic body including a base part (21) extending to an outer peripheral side of a peripheral surface of each of the through holes (11) and including at least one projecting part (22) that projects from the base part (21) and can penetrate the through hole (11); and a lid part (30) having a conductive property that clamps the base part (21) between itself and the holder part (10).

## Description

### Technical Field

The present invention relates to a brain wave detection bioelectrode, and particularly relates to a brain wave detection bioelectrode that detects a brain wave by contacting the scalp of a subject.

### Background Art

A brain wave detection bioelectrode has conventionally been used for, for example, detecting a brain wave signal for analyzing a brain function state for the purpose of detecting Alzheimer's disease in an early stage, or the like. The brain wave detection bioelectrode is used for detecting a brain wave signal by making an electrode part directly contact the scalp of a subject.
As the electrode part of the conventional brain wave detection bioelectrode, the one in which a metal electrode member is coated by an elastic member containing electrolyte and the one in which a metal electrode member including a pointed tip end part is coated by an elastic member are provided (for example, see Patent Literature 1).

### Document List

### Patent Literature

Patent Literature 1: Japanese Patent Application Publication No. 2006-34429

### Summary of Invention

### Technical Problem

In such a conventional brain wave detection bioelectrode, an electrode member is made of metal and adhesion to head scalp is low. Therefore, for the conventional brain wave detection bioelectrode, a structure that allows adhesion to be improved has been required.

The present invention has been made in view of the problem mentioned above, and it is an object of the present invention to provide a brain wave detection bioelectrode capable of improving adhesion.

### Solution to Problem

In order to achieve the above-mentioned object, a brain wave detection bioelectrode of the present invention is characterized by including: a holder part that includes a plurality of through holes; an electrode part that is formed of at least one conductive elastic body including a base part extending to an outer peripheral side of a peripheral surface of each of the through holes and including at least one projecting part that projects from the base part and can penetrate the through hole; and a lid part having a conductive property that clamps the base part between itself and the holder part.

In the brain wave detection bioelectrode according to one aspect of the present invention, the electrode part is provided for each of the plurality of through holes and each of the electrode parts includes the one projecting part.

In the brain wave detection bioelectrode according to one aspect of the present invention, the one electrode part is provided and the electrode part includes a plurality of the projecting parts each of which is provided for each of the plurality of through holes.

In the brain wave detection bioelectrode according to one aspect of the present invention, the holder part includes a step part, which is recessed in an opening direction of the through hole, so as to correspond to at least one of the electrode parts; and the step part can accommodate the base part.

In the brain wave detection bioelectrode according to one aspect of the present invention, the holder part includes a lid accommodating part that is a recessed part capable of accommodating the lid part.

### Effects of Invention

According to a brain wave detection bioelectrode of the present invention, adhesion can be improved.

### Brief Description of Drawings

[Fig. 1] A front view schematically showing the configuration of a brain wave detection bioelectrode according to a first embodiment of the present invention.
[Fig. 2] A cross-sectional view of the brain wave detection bioelectrode according to the first embodiment of the present invention in cross section A-A shown in Fig. 1.
[Fig. 3] A front view schematically showing the configuration of a brain wave detection bioelectrode according to a second embodiment of the present invention.
[Fig. 4] A cross-sectional view of the brain wave detection bioelectrode according to the second embodiment of the present invention in cross section B-B shown in Fig. 3.

### Description of Embodiments

Hereinafter, embodiments of the present invention will be described with reference to drawings.

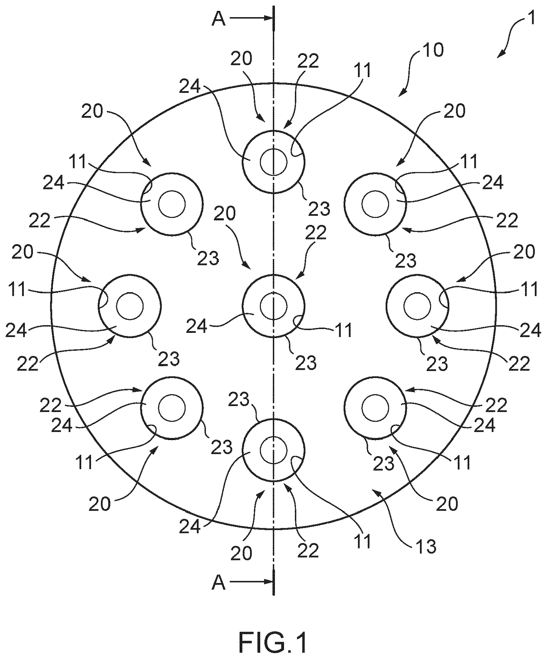

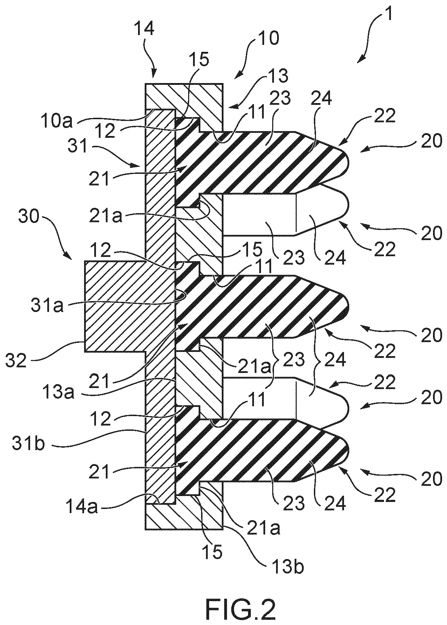

Fig. 1 is a front view schematically showing the configuration of a brain wave detection bioelectrode 1 according to a first embodiment of the present invention; and Fig. 2 is a cross-sectional view of the brain wave detection bioelectrode 1 in cross section A-A shown in Fig. 1.
As shown in Figs 1 and 2, the brain wave detection bioelectrode 1 according to the first embodiment of the present invention includes: a holder part 10 that includes a plurality of through holes 11; an electrode part 20 that is formed of at least one conductive elastic body including a base part 21 extending to an outer peripheral side of a peripheral surface of each of the through holes 11 and including at least one projecting part 22 that projects from the base part 21 and can penetrate the through hole 11; and a lid part 30 having a conductive property that clamps the base part 21 between itself and the holder part 10. Hereinafter, the configuration of the brain wave detection bioelectrode 1 will be specifically described.

The brain wave detection bioelectrode 1 is used by being connected to a signal analysis device and detects a brain wave by contact of the projecting parts 22 of the electrode parts 20 with head scalp of a subject. The detected brain wave is transmitted as an electric signal to the signal analysis device via the electrode parts 20, the lid part 30, and a lead wire which is not illustrated. Thereby, the brain wave of the subject is analyzed in the signal analysis device.

As shown in Figs 1 and 2, the holder part 10 is a member of a disk shape or a substantially disk shape, in which a lid accommodating part 10a that is a recessed part having a predetermined depth, which is formed so as to be able to accommodate the lid part 30, is formed. Specifically, the holder part 10 includes: a disk part 13 of a disk shape or a substantially disk shape; and a cylindrical part 14 of a cylindrical shape or a substantially cylindrical shape that is erected from a facing surface 13a that is one surface of the disk part 13. The cylindrical part 14 is formed at, for example, an outer edge of the disk part 13 as shown in Fig. 2. It should be noted that the arrangement position of the cylindrical part 14 in the holder part 10 is not limited thereto and the cylindrical part 14 may be formed on an inner peripheral side of the outer edge of the disk part 13 in the holder part 10. In the holder part 10, the disk part 13 and the cylindrical part 14 are integrally formed. The holder part 10 is formed of, for example, an insulator such as plastic, rubber, or the like. It should be noted that a material of the holder part 10 is not limited thereto and it may be a conductor such as a metal or the like.

In the holder part 10, the disk part 13 is provided with a plurality of through holes 11 that penetrate the disk part 13 between the facing surface 13a and an outer surface 13b that is a surface faced back-to-back with the facing surface 13a. Each of the plurality of through holes 11 is formed in a cylindrical shape or a substantially cylindrical shape. In addition, the holder part 10 includes a step part 12 recessed in an opening direction of the through hole 11, so as to correspond to each of the electrode parts 20. The step part 12 is configured so as to be able to accommodate the base part 21.

Specifically, in the disk part 13, a plurality of the step parts 12 recessed from the facing surface 13a are provided so as to respectively correspond to the plurality of through holes 11; one of the step parts 12 is provided for one of the through holes 11. The step parts 12 are cylindrical shaped or substantially cylindrical shaped, and is formed coaxially or substantially coaxially with corresponding through holes 11. In addition, the diameter of the step parts 12 is larger than the diameter of the through holes 11. A pair of a through hole 11 and a step part 12 forms one through hole with a step, and this though hole with a step forms an electrode holding hole 15 that can hold the electrode part 20. For example, one electrode part holding hole 15 is provided at a center or an substantial center of the disk part 13; in addition, a plurality of (for example, eight) electrode part holding holes 15 are provided at equal angular intervals or at substantially equal angular intervals concentrically or almost concentrically from the center or substantial center of the disk part 13. It should be noted that the arrangement positions of the electrode part holding holes 15 in the disk part 13 are not limited thereto and it is only required that the disk part 13 may include the electrode part holding holes 15 at predetermined positions.

The brain wave detection bioelectrode 1 includes one electrode part 20 corresponding to one electrode part holding hole 15 and the electrode part 20 includes the base part 21 and one projecting part 22. That is, the brain wave detection bioelectrode 1 includes a plurality of the electrode parts 20 each of which includes one base part 21 and one projecting part 22. In each of the electrode parts 20, the base part 21 and the projecting part 22 are integrally formed.

The electrode parts 20 are each formed of, for example, a conductive elastic body. The conductive elastic body is formed of, for example, an elastic body with which a conductive powder material has been mixed. The elastic body is, for example, a polymer material such as silicone resin, or the like. In addition, as the conductive power material, for example, graphite, carbon nanotube, metal particles such as silver particles, AgCl particles, or the like is used. It should be noted that the material of the electrode parts 20 is not limited thereto and the conductive elastic body may be formed by, for example, a polymer material impregnated with an organic conductive polymer or ionic liquid.

In each of the electrode parts 20, the base part 21 is formed in a disk shape or a substantially disk shape. The diameter of the base part 21 is slightly larger to an outer peripheral side than the diameter of the step parts 12 of the disk part 13, so that the base part 21 is held by being fitted into the step part 12. In addition, a value of the thickness (width in an extending direction of the projecting parts 22) of the base part 21 is slightly larger than a value of the depth (width in an extending direction of the through holes 11) of the step parts 12 of the holder part 10.

In each of the electrode parts 20, the projecting part 22 has its length in an extending direction longer than the length in an extending direction of the through holes 11 of the disk part 13, so as to penetrate the through holes 11 of the disk part 13 and project from the outer surface 13b that is faced back-to-back with the facing surface 13a of the disk part 13. The projecting parts 22 each includes: a projecting part body part 23 that is a part of a columnar shape or substantially columnar shape extending from the center or substantial center of the facing surface 21a that is one surface of the base part 21; and a brush part 24 that is a part of a cone shape or substantially cone shape. The diameter of the projecting body part 23 is identical or substantially identical to the diameter of the through holes 11 of the disk part 13; and the projecting part body part 23 is configured to be able to penetrate the through holes 11. The projecting part body part 23 is at a position coaxial or substantially coaxial with the base part 21. The length in an extending direction of the projecting part body parts 23 is preferable to be longer than the length in an extending direction of the through holes 11 of the disk part 13. The brush part 24 is connected to a tip end of the projecting part body part 23 and has a tapered shape in which its diameter gradually becomes smaller toward its tip end part. The brush part 24 is a contact part that contacts, mainly, head scalp of a subject. The brush part 24 is preferable to include its tip end part that is rounded.

The lid part 30 includes: a fixing part 31 of a disk shape or substantially disk shape; and an output part 32 of a columnar shape or substantially columnar shape that is erected coaxially or substantially coaxially with the fixing part 31. The output part 32 extends from the outer surface 31b that is a surface faced back-to-back with the facing surface 31a that is one surface of the fixing part 31. In the lid part 30, the fixing part 31 and output part 32 are integrally formed. The lid part 30 is formed by, for example, a conductor such as metal or a conductive elastic body. The diameter of the fixing part 31 is slightly larger to an outer peripheral side of the diameter of an inner surface 14a of the cylindrical part 14 of the holder part 10, so that the fixing part 31 is held by being fitted into the cylindrical part 14. That is, the fixing part 31 is fixed by being accommodated in the lid accommodating part 10a. In the lid part 30, the output part 32 is attached with a lead wire for connecting to a signal analysis device which is not illustrated.

Each of the holder part 10, the electrode parts 20, and the lid part 30 has the configuration as described above; and in the brain wave detection bioelectrode 1, each of the electrode parts 20 is fitted into corresponding each of the electrode part holding holes 15 and the lid part 30 is fitted into the lid accommodating part 10a of the holder part 10. Specifically, the projecting parts 22 of the electrode parts 20 penetrate the through holes 11 and project from the outer surface 13b of the disk part 13 of the holder part 10; and the base parts 21 are fitted into the step parts 12. Consequently, each of the electrode parts 20 is held in the holder part 10. In addition, the fixing part 31 of the lid part 30 is fixed to the holder part 10 by being fitted into the lid accommodating part 10a of the holder part 10; and further, the fixing part 31 compresses and clamps the base parts 21 of the electrode parts 20 between itself and the step parts 12. Consequently, each of the electrode parts 20 is held between the holder part 10 and the lid part 30. Thus, in the brain wave detection bioelectrode 1, the base parts 21 of the electrode part 20 are held between the lid part 30 and the holder part 10 and in the step parts 12, being firmly held.

Thus, according to the brain wave detection bioelectrode 1, the electrode parts 20 are formed of a conductive elastic body and the electrode parts 20 are easily deformed, so that when used, the whole of the electrode parts 20 easily contacts the scalp of a subject. On the other hand, the base parts 21 are also easily deformed; however, they are firmly held between the lid part 30 and the holder part 10 and in the step parts 12; and even when the electrode parts 20 are pressed against the scalp of a subject or the brain wave detection bioelectrode 1 is moved in this state and thereby an angle at which the electrode parts 20 are pressed is changed, the electrode parts 20 can be prevented from coming off the holder part 10. In addition, it is not necessary to provide the holder part 10 and the electrode parts 20 with a structure for screwing or the like and further, it is not necessary to use primer coating such as an adhesive between the holder part 10 and the electrode parts 20; and therefore, its structure can be simplified and miniaturization can be achieved. Especially, also the brain wave detection bioelectrode 1 can be miniaturized in its extending direction (direction orthogonal to a paper surface in Fig. 1). In addition, adhesion between the lid part 30 and the electrode part 20 is improved and therefore, it can be effectively prevented that transmission of a brain wave signal is inhibited.

Further, each of the electrode parts 20 is provided for each of the plurality of through holes 11 and each of the electrode parts 20 includes one projecting part 22 and therefore, even when an electrode part 20 including a projection part 22 of a different diameter and shape is desired to be used or any projecting part 22 is broken, the electrode part 20 can be easily replaced in units of the electrode parts 20 and therefore, maintainability and usability can be improved.

In addition, as described above, the electrode parts 20 are each formed of a conductive elastic body and its flexibility and elasticity makes the adhesion to the scalp of a subject excellent and therefore it provides a soft touch feeling such that even when the scalp of a subject is adhered for a long time, the subject hardly feels uncomfortable.

Thus, in the brain wave detection bioelectrode 1 according to the first embodiment of the present invention, adhesion can be improved.

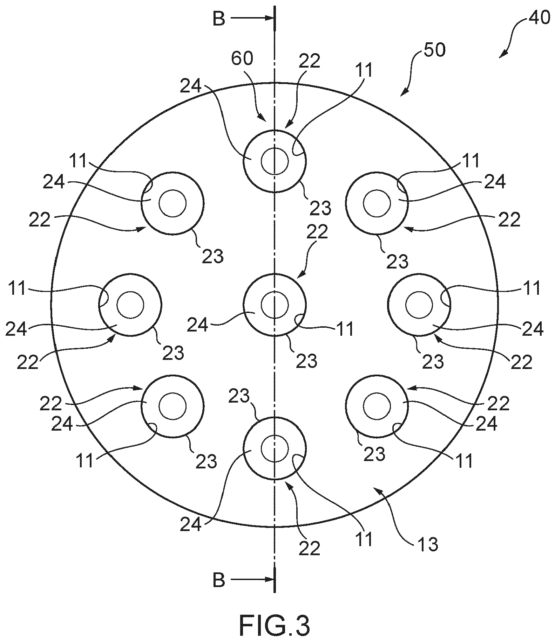

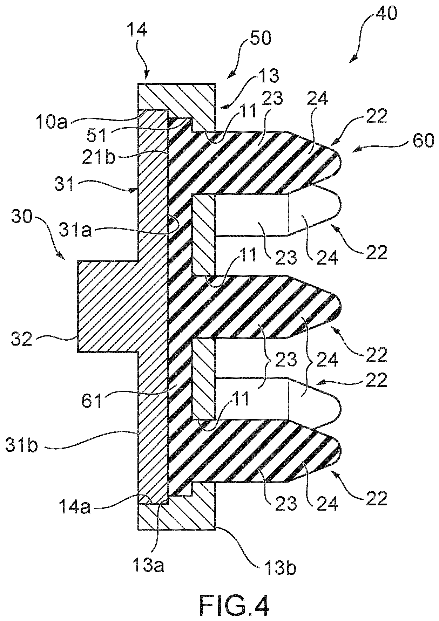

Next, the configuration of a brain wave detection bioelectrode 40 according to a second embodiment of the present invention will be described. Fig. 3 is a front view schematically showing the configuration of a brain wave detection bioelectrode 40; and Fig. 4 is a cross-sectional view of the brain wave detection bioelectrode 40 in cross section B-B shown in Fig. 3. Hereinafter, a component identical or similar to the one of the brain wave detection bioelectrode 1 according to the first embodiment described above will be denoted by the same reference sign and description thereof will be omitted; and only different components will be described. The brain wave detection bioelectrode 40 according to the second embodiment is different from the brain wave detection bioelectrode 1 according to the first embodiment in the configurations of the holder part and electrode parts. Specifically, instead of the step parts 12 of the holder part 10 and the base parts 21 of the electrode parts 20, a step part 51 is provided in the holder part 50 and a base part 61 is provided on an electrode part 60.

The brain wave detection bioelectrode 40 includes, as shown in Figs. 3 and 4, one electrode part 60; and the electrode part 60 includes a plurality of projecting parts 22 which are individually provided for each of a plurality of through holes 11. In addition, in the holder part 50, only one step part 51 is provided for the plurality of through holes 11. Specifically, in the disk part 13, one step part 51 that is recessed from the facing surface 13a is provided. The step part 51 is disk shaped or substantially disk shaped, and is formed coaxially or substantially coaxially with the disk part 13. In addition, the diameter of the step part 51 is smaller than the diameter of the inner surface 14a of the cylindrical part 14. The through holes 11 are positioned so as to be within a range of the step part 51.

The base part 61 of the electrode part 60 corresponds to the step part 51 and is formed in a disk shape or a substantially disk shape. The diameter of the base part 61 is slightly larger to an outer peripheral side than the diameter of the step part 51 of the holder part 50, so that the base part 61 is held by being fitted into the step part 51. In addition, a value of the thickness (width in an extending direction of the projecting parts 22) of the base part 61 is slightly larger than a value of the depth (width in an extending direction of the through holes 11) of the step part 51 of the holder part 50.

The projecting parts 22 are arranged on the base part 61 so as to obtain a relative position relation similar to the relative position relation of the through holes 11 in the disk part 13. In this embodiment, one thereof is provided in a center or substantial center of the base part 61 so as to correspond to one of the through holes 11 that is in a center or substantial center of the disk part 13; and a plurality (for example, eight) thereof are provided concentrically or substantially concentrically from the center or substantial center of the base part 61 so as to correspond to the plurality of (for example, eight) through holes 11 which are arranged concentrically or substantially concentrically from the center or substantial center of the disk part 13. It should be noted that the arrangement positions of the projection parts 22 in the base part 61 are not limited thereto and it is only required that a predetermined number of projecting part 22 which corresponds to that of the through holes 11 are provided at predetermined positions corresponding to those of the through holes 11 in the base part 61.

The brain wave detection bioelectrode 40 having the configuration described above can improve adhesion and in addition, can exert effects similar to those of the brain wave detection bioelectrode 1 described above. In addition, in the brain wave detection bioelectrode 40, the one electrode part 60 includes the plurality of projecting parts 22 which are individually provided for each of the plurality of through holes 11; and therefore, each of the projecting parts 22 is more firmly held between the holder part 50 and the lid part 30 and adhesion to the scalp of a subject can be made further excellent. Further, the electrode part 60 can be attached to the holder part 50 at a time and therefore, the structure of the brain wave detection bioelectrode 40 can be simplified and its manufacture can be made easy.

Although the above has described the embodiments of the present invention, the present invention is not limited to the above embodiments of the present invention and includes any aspects that are included in the concepts and the scope of claims of the present invention. In addition, the configurations may be appropriately combined as appropriate so as to produce an effect for at least part of the problem and effects described above. For example, the shape, material, arrangement, size, and the like of each of the components in the above embodiments can be appropriately changed according to the specific usage mode of the present invention.

For example, the embodiments of the present invention have been described as the brain wave detection bioelectrodes 1, 40 by using, as examples, cases in which the projecting parts 22 each includes the projecting part body part 23 and the brush part 24. However, the projecting parts 22 may be formed in a cone shape or substantially cone shape as a whole; alternatively, it may be formed in other shapes such as a columnar shape, a prism shape, or the like.

In addition, the embodiments of the present invention have been described as brain wave detection bioelectrodes, by using, as examples, a mode in which the step parts 12 and 51 are formed in a cylindrical shape or substantially cylindrical shape or in a disk shape or substantially disk shape and the bottom parts 21 and 61 are formed in a disk shape or substantially disk shape. However, the step parts 12 and 51 may be in a quadrangular cylindrical shape and the bottom parts 21 and 61 may be in a quadrangular prism shape respectively corresponding to the step parts 12 and 51 and their shapes are not limited.

In addition, the embodiments of the present invention have been described as brain wave detection bioelectrodes, by using, as examples, a case in which the inner surface 14a of the cylindrical part 14 is formed in a cylindrical surface shape or substantially cylindrical surface shape or is in a disk shape or substantially disk shape, and the fixing part 31 of the lid part 30 is formed in a disk shape or substantially disk shape. However, the inner surface 14a of the cylindrical part 14 may be in a quadrangular cylindrical surface shape and the fixing part 31 may be in a quadrangular columnar shape corresponding to the inner surface 14a of the cylindrical part 14 and their shapes are not limited.

In addition, in the brain wave detection bioelectrodes 1 and 40 according to the embodiments described above, the step parts 12 or 51 into which the base part 21 or 61 of the electrode part 20 or 60 is fitted are formed in the disk part 13 of the holder part 10 or 50; however in the disk part 13, the step parts 12 and 51 do not need to be formed. This is because, also in this case, the base part 21 or 61 of the electrode part 20 or 60 is clamped between the facing surface 13a of the disk part 13 and the facing surface 31a of the fixing part 31 of the lid part 30, so that the electrode part 20 or 60 can be fixed between the holder part 10 or 50 and the lid part 30.

In addition, in the brain wave detection bioelectrodes 1 and 40 according to the embodiments described above, the fixing part 31 of the lid part 30 is fitted into the lid accommodating part 10a of the holder part 10 or 50, so that the lid part 30 is fixed to the holder part 10 or 50; however, a configuration for fixing the lid part 30 to the holder part 10 or 50 is not limited to this fitting. The lid part 30 may be fixed to the holder part 10 or 50 with a bolt and a screw hole or nut, or may be fixed with other fixing members. In this case, the lid accommodating part 10a may be omitted in the holder part 10 or 50.

### List of Reference Signs

1, 40 brain wave detection bioelectrode,
10, 50 holder part,
10a lid accommodating part,
11 through hole,
12, 51 step part,
13 disk part,
13a facing surface,
13b outer surface,
14 cylindrical part,
14a inner surface,
15 electrode part holding hole,
20, 60 electrode part,
21, 61 base part,
21a facing surface,
22 projecting part,
23 projecting part body part,
24 brush part,
30 lid part,
31 fixing part,
31a facing surface,
31b outer surface,
32 output part

## Claims

1. A brain wave detection bioelectrode, **characterized in** comprising:
a holder part including a plurality of through holes;
an electrode part formed of at least one conductive elastic body, the conductive elastic body including a base part and at least one projecting part, the base part extending to an outer peripheral side of a peripheral surface of each of the through holes, the projecting part projecting from the base part and capable of penetrating the through hole; and
a lid part having a conductive property, the lid part clamping the base part between itself and the holder part.

2. The brain wave detection bioelectrode according to claim 1, **characterized in that**:
each of a plurality of the electrode parts is provided for each of the plurality of through holes and each of the electrode parts includes the one projecting part.

3. The brain wave detection bioelectrode according to claim 1, **characterized in** comprising:
the one electrode part, the electrode part including a plurality of the projecting parts which are respectively provided for the plurality of through holes.

4. The brain wave detection bioelectrode according to any one of claims 1 to 3, **characterized in that**:
the holder part includes a step part, which is recessed in an opening direction of the through holes, so as to correspond to the at least one electrode part; and the step part can accommodate the base part.

5. The brain wave detection bioelectrode according to any one of claims 1 to 4, **characterized in that**:
the holder part includes a lid accommodating part, the lid accommodating part being a recessed part capable of accommodating the lid part.
